# EUROPEAN PATENT APPLICATION

(11) **EP 2 799 873 A2**
(43) Date of publication of application: **05.11.2014**
(21) Application number: 14163559.9
(22) Date of filing: 04.04.2014
(51) Int. Cl.: G01N 33/543, G01N 33/579

(54) **Detection and/or quantitation of endotoxin**

(30) Priority: 29.04.2013 US 201313872433
(71) Applicant: Pall Corporation, Port Washington, NY 11050 (US)
(72) Inventor: Witte, Krista L., Hayward, CA California 94542 (US); Harper, Theresa F., Castro Valley, CA California 94546 (US); Egholm, Michael, Mamaroneck, NY New York 10543 (US); Gsell, Thomas, Glen Head, NY New York 11545 (US); Angeles, Angel N., Union City, CA California 94587 (US); Wicke, Robert H., San Francisco, CA California 94117 (US)
(74) Representative: Hoeger, Stellrecht & Partner Patentanwälte

(57) **Abstract**

Detection and/or quantitation of endotoxin using biolayer interferometry is disclosed.

## Description

### BACKGROUND OF THE INVENTION

Bacterial endotoxins can be produced, secreted and/or released from Gram-negative bacteria. Bacterial endotoxins can contaminate a variety of materials such as water, food, pharmaceutical products, and parenteral preparations. Bacterial endotoxins can be dangerous, and sometimes deadly, to humans. Manufacturers in the pharmaceutical, medical device, food, and beverage industries, among other industries, must meet standards to verify that their products do not contain levels of contaminants that would compromise the health of the recipient or consumer. For example, certain manufacturers of pharmaceuticals and medical devices may have to establish that their products are free of detectable levels of Gram negative bacterial endotoxins. Additionally, in order to promptly initiate proper medical treatment for patients possibly infected with Gram negative bacteria, it is important to determine, as early as possible, whether endotoxin is present, and, if possible, the concentration of the endotoxin in the patient.

Typical assays for endotoxin use *Limulus amebocyte* lysate (LAL), wherein the LAL is exposed to an endotoxin, leading to a clotting cascade producing a product that is analyzed via a gel clot assay, an end-point turbidometric assay, a kinetic turbidometric assay, or an endpoint chromogenic assay.

However, there is a need for improved methods and systems for detecting the presence and/or concentration of endotoxin.

### BRIEF SUMMARY OF THE INVENTION

An embodiment of the invention provides a method of assaying a sample for endotoxin, the method comprising (a) placing a sample to be assayed in contact with LAL reagent, wherein endotoxin, if present in the sample, reacts with the reagent and a clotted product is formed; (b) contacting a tip of an optical fiber with the sample, wherein clotted product, if present, forms on and/or binds to the tip of the optical fiber, wherein the tip of the optical fiber has an optical thickness, and the optical thickness increases from an initial thickness when clotted product forms on and/or binds to the tip of the optical fiber; (c) determining the optical thickness at the tip of the optical fiber after contact with the sample, wherein an increase in optical thickness from the initial thickness indicates the presence of endotoxin, and an absence of an increase in optical thickness indicates the absence of endotoxin. In some embodiments, the method comprises determining a concentration of endotoxin in the sample.

In some embodiments, the method includes contacting tips of a plurality of optical fibers, and determining the optical thicknesses at the tips of the optical fibers after contact with the sample. In those embodiments of the method including a plurality of optical fibers, fibers can be utilized individually and/or the fibers may be arranged in a bundle.

In another embodiment, a kit for assaying a sample for endotoxin is provided, the kit comprising a biosensor comprising an optical fiber having a tip, and LAL reagent.

In yet another embodiment, a kit for assaying a sample for endotoxin is provided, the kit comprising a housing, the housing comprising a base and a cap, the base comprising a groove, and a reaction chamber having a side wall, the chamber having an aperture passing through the side wall, the aperture communicating with the groove; the cap comprising a sample receiving chamber, and a projection including a sidewall and at least one aperture passing through the projection side wall, wherein the cap is removably engageable with the base. In a more preferred embodiment, the reaction chamber has LAL reagent therein, and/or an optical fiber is arranged in the groove of the base, wherein the optical fiber tip communicates with the reaction chamber through the aperture.

A kit for assaying a sample for endotoxin according to another embodiment of the invention comprises a biosensor comprising an optical fiber having a tip, LAL reagent, and a housing, the housing comprising a base and a cap, the base comprising a groove, and a reaction chamber having a side wall, the chamber having an aperture passing through the side wall, the aperture communicating with the groove; the cap comprising a sample receiving chamber, and a projection including a sidewall and at least one aperture passing through the projection side wall, wherein the cap is removably engageable with the base, and wherein the reaction chamber has LAL reagent therein, and the optical fiber is arranged in the groove of the base, wherein the optical fiber tip communicates with the reaction chamber through the aperture.

### BRIEF DESCRIPTION OF THE SEVERAL VIEWS OF THE DRAWING(S)

Figures 1A and 1B show illustrative optical assemblies suitable for use in an assay according to an embodiment of the present invention.
Figure 2 shows a dose response with different endotoxin concentrations in an assay according to an embodiment of the present invention.
Figure 3 shows a dose response with different endotoxin concentrations in an assay using different biosensors and different sample chambers according to embodiments of the present invention. Figures 3A and 3B show Aminopropylsilane (APS) functionalized biosensors with a polystyrene sample chamber and a polypropylene sample chamber, respectively; Figures 3C and 3D show SiO₂ functionalized biosensors with a polystyrene sample chamber and a polypropylene sample chamber, respectively.
Figure 4 shows a dose response with different endotoxin concentrations in an assay including shaking the sample (Figure 4A), and without shaking the sample (Figure 4B), according to embodiments of the present invention.

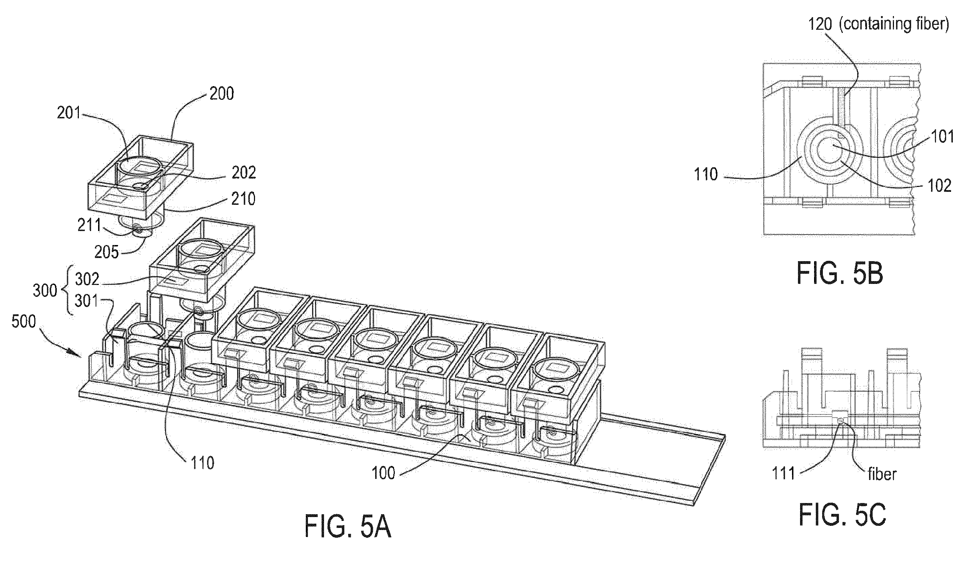

Figure 5 shows a kit according to an embodiment of the present invention, illustrating a housing comprising a base and a cap, the base comprising a groove, and a reaction chamber having a side wall, the chamber having an aperture passing through the side wall; the cap comprising a sample receiving chamber, and a projection including a sidewall and at least one aperture passing through the projection side wall, wherein the cap is removably engageable with the base. Figure 5A shows a perspective and partially exploded view, Figures 5B and 5C show, respectively, top and front views of the base, also showing an optical fiber having a tip, arranged in the groove of the base, wherein the tip communicates with the reaction chamber through the aperture.

### DETAILED DESCRIPTION OF THE INVENTION

In accordance with an embodiment of the present invention, method of assaying a sample for endotoxin is provided, the method comprising (a) placing a sample to be assayed in contact with LAL reagent, wherein endotoxin, if present in the sample, reacts with the reagent and a clotted product is formed; (b) contacting a tip of an optical fiber with the sample, wherein clotted product, if present, forms on and/or binds to the tip of the optical fiber, wherein the tip of the optical fiber has an optical thickness, and the optical thickness increases from an initial thickness when clotted product forms on and/or binds to the tip of the optical fiber; (c) determining the optical thickness at the tip of the optical fiber after contact with the sample, wherein an increase in optical thickness from the initial thickness indicates the presence of endotoxin, and an absence of an increase in optical thickness indicates the absence of endotoxin.

In some embodiments, the method comprises determining a concentration of endotoxin in the sample.

In some embodiments, the method includes contacting tips of a plurality of optical fibers, and determining the optical thicknesses at the tips of the optical fibers after contact with the sample. In those embodiments of the method including a plurality of optical fibers, fibers can be utilized individually and/or the fibers may be arranged in a bundle.

In one embodiment, a kit for assaying a sample for endotoxin is provided, the kit comprising a biosensor comprising an optical fiber having a tip, and LAL reagent.

In another embodiment, a kit for assaying a sample for endotoxin is provided, the kit comprising a housing, the housing comprising a base and a cap, the base comprising a groove, a reaction chamber having a side wall, the chamber having an aperture passing through the side wall, the aperture communicating with the groove; the cap comprising a sample receiving chamber, and a projection including a sidewall and at least one aperture passing through the projection side wall, wherein the cap is removably engageable with the base. In a more preferred embodiment, the reaction chamber has LAL reagent therein, and/or an optical fiber is arranged in the groove of the base, wherein the optical fiber tip communicates with the reaction chamber through the aperture.

In another embodiment, a kit for assaying a sample for endotoxin is provided, the kit comprising a biosensor comprising an optical fiber having a tip, LAL reagent, and a housing, the housing comprising a base and a cap, the base comprising a groove, a reaction chamber having a side wall, the reaction chamber having an aperture passing through the side wall, the aperture communicating with the groove; the cap comprising a sample receiving chamber, and a projection including a sidewall and at least one aperture passing through the projection side wall, wherein the cap is removably engageable with the base, and wherein the reaction chamber has LAL reagent therein, and the optical fiber is arranged in the groove of the base, wherein the optical fiber tip communicates with the reaction chamber through the aperture.

In preferred embodiments of the kits, the kits include a plurality of plurality of optical fibers; more preferably, wherein the kits include a plurality of reaction chambers and each chamber includes at least one optical fiber.

Conventional systems for detecting endotoxin suffer from one or more of the following drawbacks, e.g., determining whether a clot has formed can be a subjective decision and/or disturbing the sample can prevent the clot from forming or destroy the clot; turbidity measurements can be impacted by the presence of impurities in the sample or the sample chamber; the timing of reagent addition and start of detection must be carefully timed, colorimetric labels and/or secondary dyes are needed; a change in substrate color must be measured, and/or only end-point measurement is possible, rather than real-time monitoring.

In contrast, in accordance with the invention, a reproducible and reliable label-free assay is provided, requiring minimal manual steps by the user, with controlled timing. There is no need to measure turbidity or substrate color. The assay is suitable for use with both Gel-clot and turbidimetric-type LAL reagents. Additionally, quantitative real-time data can be obtained rapidly.

In accordance with the invention, the presence and/or quantity of endotoxin in a sample is determined using biolayer interferometry (BLI), a label-free technology, involving an analysis of white light reflected from two surfaces: the surface of a biosensor tip (which may include a bound material forming a layer), and an internal reference layer. Binding of material or molecules at the biosensor tip will increase the optical thickness at the tip, resulting in a wavelength shift, which is a direct measure of the change in thickness of the layer at the tip. Unbound material or molecules, changes in the refractive index of the surrounding medium and/or changes in flow rate do not affect the thickness of the layer at the tip. Traditionally, BLI has been used with analyte and analyte-binding molecules, wherein one type of molecule (e.g., the analyte-binding molecule) was immobilized on the biosensor tip, and the biosensor tip was placed in a sample volume, and the other type of molecule (e.g., the analyte molecule), if present in the sample volume, specifically binds to the immobilized molecule, increasing the thickness of the layer at the tip.

However, the inventors of the present invention surprisingly found that BLI can be used in an endotoxin assay, without analyte and analyte-binding molecules, and without immobilizing one type of molecule on the tip for specifically binding the other type of molecule in the sample. Rather, in an endotoxin assay, the clotting cascade product can be formed separate from and independent of the biosensor tip, and the product subsequently binds to the biosensor tip and/or the clotting cascade product can be formed on the biosensor tip.

Embodiments of the invention can be used in a variety of applications, e.g., for testing endotoxin contamination in at-line, near-line, laboratory, and hospital, settings, and are particularly useful for manufacturers in the pharmaceutical, medical device, food, beverage, and electronics industries, among other industries.

A wide variety of samples can be analyzed according to embodiments of the invention, and sources of the samples range from, for example, water, incoming raw materials (such as, but not limited to, urine, and starting materials used for the preparation of pharmaceutical products), and biological samples. Biological samples include biological fluids, that include any treated or untreated fluid associated with living organisms, urine, cerebrospinal fluid, ascites fluid, saliva, and blood, including whole blood, cord blood, and stored or fresh blood; treated blood, such as blood diluted with at least one physiological solution, including but not limited to saline, nutrient, and/or anticoagulant solutions; blood components, such as platelet concentrate (PC), platelet-rich plasma (PRP), platelet-poor plasma (PPP), platelet-free plasma, plasma, fresh frozen plasma (FFP), components obtained from plasma, packed red cells (PRC), buffy coat (BC); blood products derived from blood or a blood component or derived from bone marrow; stem cells; red cells separated from plasma and resuspended in a physiological solution or a cryoprotective fluid; and platelets separated from plasma and resuspended in a physiological solution or a cryoprotective fluid. As used herein, blood product or biological fluid refers to the components described above, and to similar blood products or biological fluids obtained by other means and with similar properties. Biological samples as described above can also include as an additional component, one or more of the following, for example: EDTA and heparin (e.g., a biological sample comprising plasma can also include EDTA and/or heparin).

The following definitions are used in accordance with the invention.

LAL reagent. An LAL reagent includes any ameboid lysate obtained from a crustacean such as a horseshoe crab (e.g., *Limulus spp., Tachypleus spp.,* and *Carcinoscorpius spp*.) as well as synthetic LAL reagents. A variety of LAL reagents are suitable for use in the invention, and suitable LAL reagents are known in the art. Suitable commercially available LAL reagents are available from, for example, Charles River Laboratories, Associates of Cape Cod, Inc., Wako Chemicals USA, and Lonza. LAL reagents are typically lyophilized, and reconstituted with endotoxin-free water or low endotoxin water (LEW) (sometimes referred to as "LAL reagent water").

Biolayer interferometry (BLI). As note above, BLI is a label-free technology, involving an analysis of white light reflected from two surfaces: the surface of a biosensor tip (which may include a bound material forming a layer), and an internal reference layer. Binding of material or molecules at the biosensor tip will increase the optical thickness at the tip, resulting in a wavelength shift, which is a direct measure of the change in thickness of the layer at the tip. BLI technology used in accordance with embodiments of the invention is disclosed in, for example, U.S. Patents 5,804,453 and 7,394,547, as well as U.S. Patent Application Publication 2010/0093106 and International Publication WO 2006/138294. A variety of chemistries are suitable for use in providing biosensor functionalized tips according to embodiments of the invention.

A variety of materials are suitable for use in containing the sample volume, e.g., in a reaction chamber or reaction vessel, sample container, or a kit comprising the reaction chamber, the reaction vessel, and/or the sample container. If desired, the materials may also be suitable for containing, for example, LAL reagent and/or LAL free water. Materials usable in accordance with the invention (e.g., for forming the container, chamber and/or vessel) are typically polymers or glass, and include, for example, polystyrene, polypropylene, polyethylene, polyvinyl acetate, polycarbonate, glass (including borosilicate glass) and polytetrafluoroethylene (PTFE). In some embodiments, the container, chamber, and/or reaction vessel is coated with the material (or coated to, for example, reduce non-specific binding, e.g., coated with polyethylene glycol (PEG). Suitable materials and coatings, which include commercially available materials and coatings, are known in the art.

As will be discussed below, in some embodiments, it may be desirable that the surface of the biosensor tip (e.g., the chemistry at the tip) be more hydrophilic than the surface of the reaction vessel.

The assay can be carried out in a variety of formats, for example, the sample container, reaction chamber and/or reaction vessel can comprise a tube, cuvette, cartridge, well, or support such as a multiwall plate (e.g., a 96- or 384-well plate). A sample can be tested for more than one application, e.g., for research laboratory use and/or medical device testing, and/or pharmaceutical testing.

Embodiments of the invention can be carried out with a static fluid sample or a flowing fluid sample.

The sample can be any suitable volume. While a typical sample volume is in the range of from about 4 µL to about 250 µL, higher or lower volumes can be used.

Assays can be carried out at any suitable temperature, typically, the temperature range is from about 30°C to about 45°C, and in some embodiments, the preferred temperature is 37±0.5/1°C.

Assays can be carried out at any suitable pH, typically, the pH range is from about 6 to about 8.

Embodiments of the invention are compatible with a variety of methods of data analysis, including, for example, time to threshold, sigmoidal fit (determination of EC50 and Hill slope), area under curve (AUC), first derivative, determination of the lag time, and modified Ct algorithm.

Embodiments of the invention can be carried out using a variety of BLI-based commercial instruments and systems, and including single sensor and multiple sensor (e.g., 16 sensor) instruments and systems, which can be either non-switching, or, more preferably, switching, instruments and systems. Suitable BLI-based commercially available instruments and systems include, for example, instruments available from fortéBIO® (a division of Pall Life Sciences, Port Washington, NY) under the OCTET® instrument platform (e.g., OCTET® QK384/RED384, OCTET® RED96, OCTET® QKe/QK) as well as the BLITZ™ system.

Typically, a BLI-based system for use in accordance with embodiments of the invention comprises a light source, an optical assembly comprising a biosensor, a first optical waveguide or fiber that extends from the light source to the optical assembly, a second optical waveguide or fiber which carries reflected light from the optical assembly to a detector unit for detecting interference signals produced by interfering light waves reflected from the optical assembly, and an optical coupler which optically couples the fibers. Light from the light source is directed through the optical assembly, and reflected back to the detector through an optical coupling assembly. Suitable light sources, detector units, optical fiber and coupling components are known in the art.

Each of the components of the invention will now be described in more detail below, wherein like components have like reference numbers.

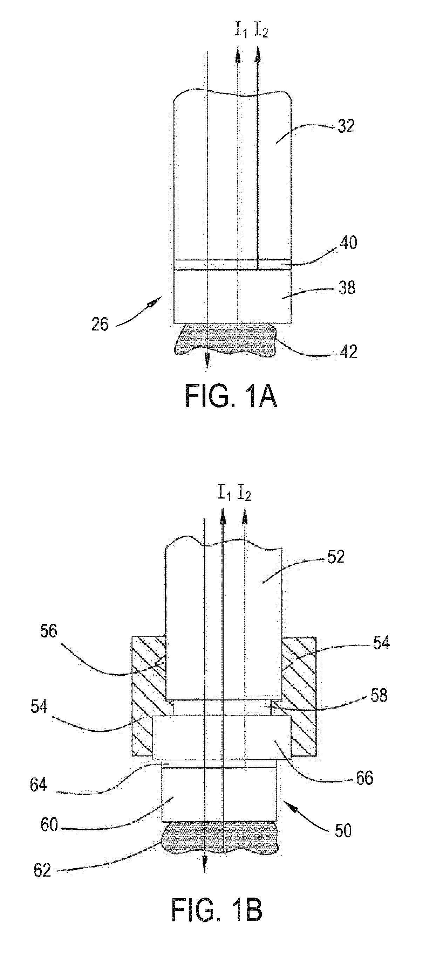

Figure 1A shows an exemplary optical assembly comprising a biosensor 26, and an adjoining portion of the distal region of a first optical waveguide or fiber 32 extending from a light source to the biosensor, wherein the fiber is fixedly attached to the biosensor. The illustrated biosensor comprises a transparent optical element 38 having first and second reflecting surfaces 42, 40 formed on its lower (distal) and upper (proximal) end faces, respectively. The first reflective surface 42 surface is formed of a layer of clotting cascade product. The second reflective surface 40 is formed as a layer of transparent material having an index of refraction that is substantially higher than that of the optical element 38, such that this layer functions to reflect a portion of the light directed onto the biosensor. The measurement of the presence, concentration and/or binding rate of the clotted cascade product to the biosensor is enabled by the interference of reflected light beams from the two reflecting surfaces in the biosensor. Because the thickness of all other layers remains the same, the interference wave formed by the light waves reflected from the two surfaces is phase shifted in accordance with the thickness change.

Figure 1B shows another exemplary optical assembly comprising a biosensor 50, that is removably carried on the distal end of a first optical waveguide or fiber 52. The illustrated biosensor comprises a transparent optical element 60 having first and second reflecting surfaces 62, 64, corresponding to first and second reflecting surfaces 42, 40 shown in Figure 1A. The illustrated biosensor further includes a second optical element 66 having a thickness that is typically greater than 100 nm, preferably greater than about 200 nm, wherein layer 64 (preferably having a thickness of less than about 30 nm) is sandwiched between the optical elements 60 and 66 as shown. Figure 1B also flexible gripping arms 54, designed to slide over the end of the fiber and grip the fiber by engagement of an annual rim or detente 56 on the fiber with complementary recesses formed in the arms. This attachment can position the biosensor on the fiber to provide an air gap 58 between the distal end of the fiber and the confronting (upper) of the optical assembly, typically, an air gap of about 100 nm or less or about 2 micrometers or greater.

A thickness "d" of the biosensor between its distal and proximal surfaces, i.e., between the two reflecting surfaces illustrated in Figures 1A and 1B, is typically at least 50 nm, and in some embodiments, at least 100 nm (e.g., in the range of from about 100 to about 5,000 nm, more typically, about 400 to about 1,000 nm). Exemplary indices of refraction and materials for forming the biosensors include those disclosed in, for example, U.S. Patents 5,804,453 and 7,394,547, as well as U.S. Patent Application Publication 2010/0093106 and International Publication WO 2006/138294.

In some embodiments (not shown) the biosensor comprises a disposable detector tip (e.g., as disclosed in International Publication WO 2006/138294) that can be connected to a tip connector (that may comprise a bundle of fibers), wherein the disposable detector tip comprises an optical fiber section (having a proximal end and a distal end, the proximal end coupling to the fiber(s) in a tip connector, the distal end including the two reflecting surfaces discussed above) and a connector structure, and the assay system is designed to two or more disposable tips (e.g., 8 tips) at once. Such a system can include an optical coupling assembly implementing a collection of fiber assemblies and connectors, and can allow different types of coupling assemblies to be used with the same light source and/or detector unit, and allows different types of detector tips to be used with the same optical coupling assembly.

Figure 5 illustrates an exemplary kit 1000 for use in the invention, the kit comprising a housing 500, the housing comprising a base 100 and a cap 200, the base 100 comprising a groove 120, a reaction chamber 101 (illustrated as an annular chamber) having a side wall 110, the chamber having an aperture 111 passing through the side wall, the aperture communicating with the groove; the cap 200 comprising a sample receiving chamber (illustrated as an annular chamber) 201, a port 202, and a projection 205 including a sidewall 210 and at least one aperture 211 passing through the projection side wall, wherein the cap is removably engageable with the base. In the illustrated embodiment, the base has a collar 102 surrounding the sample receiving chamber 201 wherein the inner diameter of the collar generally corresponds to the outer diameter of the projection in the cap.

Preferably, as shown in Figure 5B (top view of base) and 5C (front view of base), an optical fiber of the biosensor is arranged in the groove of the base, wherein the optical fiber tip communicates with the reaction chamber through the aperture. In some embodiments, the kit further comprises LAL reagent in the reaction chamber, e.g., so that the user can add sample to the kit via the sample receiving chamber in the cap, wherein the sample passes from the sample receiving chamber, through the port and the aperture in the projection side wall, into the sample receiving chamber, where it contacts the LAL reagent, and the clotting cascade product forms on and/or binds to the tip of the optical fiber of the biosensor.

In the illustrated embodiment, the kit further comprises a retaining assembly 300 for engaging the cap with, and releasing the cap from, the base. Typically, the retaining assembly comprises at least one arm (two arms 301 having bent tips are illustrated as part of the base), and at least one slot (two slots 302 are illustrated as part of the cap) wherein the arm(s) pass through the slot(s) and the tip(s) of the arm(s) engage with a planar surface of the cap(s). Preferably, the retaining assembly is configured so that the cap can only be engaged with the base when the cap and base are oriented correctly. For example, in the illustrated embodiment, the arms and slots are offset, so that the cap and base cannot be misoriented by 180°.

The kit can be provided as part of a sterile package, e.g., in a hard case and/or a blister pack.

A variety of reaction chambers, reaction vessels, sample containers, and/or kits comprising reaction chambers, reaction vessels, and/or sample containers, are suitable for use in the invention.

The following examples further illustrate the invention but, of course, should not be construed as in any way limiting its scope. In the following experiments, the known endotoxin concentration standards are created using Control Standard Endotoxin (CSE) or Reference Standard Endotoxin (RSE) and Low Endotoxin Water (LEW). Unless noted otherwise in the Examples, the well reaction volumes are 80 µL, containing 320 µg lyophilized LAL reagent; the control is a blank of LEW without endotoxin; and the assays are carried out at 37° C, and at a pH of 6.3.

The samples in Examples 1, 3-6, and 8, are analyzed via BLI using an OCTET® QKe instrument (fortéBIO® a division of Pall Life Sciences, Port Washington, NY).

### EXAMPLE 1

This example demonstrates a variety of endotoxin concentrations can be determined in an assay of a sample in accordance with an embodiment of the invention.

A biosensor having an SiO₂ functionalized tip is prepared as generally described in U.S. Patent 7,394,547.

Endotoxin concentrations of 50, 5, 0.5, 0.05, 0.005, 0.0005, and 0 EU/mL are prepared. Lyophilized LAL reagent (Associates of Cape Cod Inc.; East Falmouth, MA) is resolubilized with endotoxin-free water and placed in contact with the RSE in wells of a 384 well polypropylene plate that is agitated at 1000 rpm.

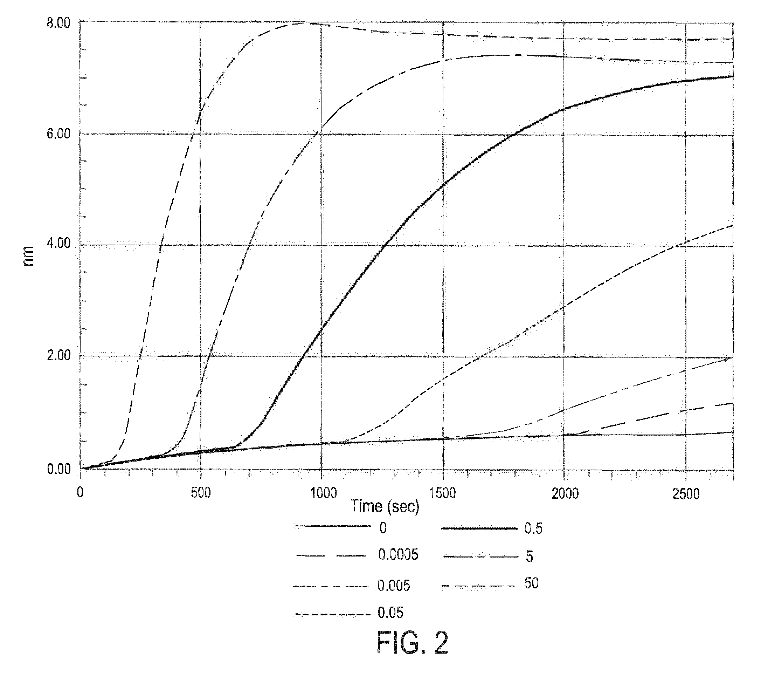

The samples are analyzed via BLI, and the results are shown in Figure 2.

This example shows the sensitivity of the assay down to 0.005 EU/ml.

### EXAMPLE 2

This example measures the refractive index (RI) upon clotting, with different concentrations of endotoxin, using a refractomer (model 334610; Spectronic Instruments, Inc., Rochester, NY).

Solutions are mixed in 1.5 mL endotoxin free microcentrifuge tubes, and 20 µL of each sample are pipetted onto the instrument's sample stage. The results are as follows.

| solution | RI | Temperature (°C) |
|---|---|---|
| H₂O | 1.332 | 24 |
| H₂O+LAL reagent | 1.333 | 24 |
| H₂O+LAL reagent+Endotoxin @ 0.5 EU/mL | 1.333 | 24 |
| H₂O+LAL reagent+Endotoxin @ 5 EU/mL | 1.333 | 24 |
| H₂O+LAL reagent+Endotoxin @ 50 EU/mL | 1.333 | 24 |
| H₂O+LAL reagent+Endotoxin @ 3000 EU/mL | 1.333 | 24 |

This example demonstrates that there is no change in the refractive index (RI) upon clotting, even with different concentrations of endotoxin ranging from to 3000 EU/mL, and thus the BLI signal is not due to RI changes.

### EXAMPLE 3

This example demonstrates that, in comparison to a conventional Gel-clot assay, a BLI assay using Gel-clot reagents carried out in accordance with an embodiment of the invention has a lower limit of detection.

A biosensor having an SiO₂ functionalized tip is prepared as generally described in U.S. Patent 7,394,547.

Endotoxin concentrations of 50, 5, 0.5, 0.05, 0.005 and 0 EU/mL are prepared. Lyophilized LAL reagent (Associates of Cape Cod Inc.; East Falmouth, MA) is resolubilized with endotoxin-free water and placed in contact with the endotoxin in wells of a 384 well polypropylene plate that is agitated at 1000 rpm.

Gel-clot assays (conventional), and BLI assays (using Gel-clot reagents) in accordance with an embodiment of the invention, are carried out with endotoxin concentrations of 50, 5, 0.5, 0.05, 0.005 and 0 EU/mL. A clot forms in the presence of a detectable concentration of endotoxin.

The conventional Gel-clot assay shows, after an assay time of 60 minutes, retained clots at endotoxin concentrations of 50, 5, and 0.5 EU/mL. The assay shows an incomplete clot at an endotoxin concentration of 0.05 EU/mL, and no clot at endotoxin concentrations of 0.005 and 0 EU/mL.

The BLI assay using Gel-clot reagents carried out in accordance with an embodiment of the invention shows, after an assay time of 34 minutes, clotting at endotoxin concentrations of 50, 5, 0.5 and 0.05 EU/mL. The assay shows a slight signal at an endotoxin concentration of 0.005 and no signal at an endotoxin concentration of 0 EU/mL.

This example shows that, in contrast with a conventional Gel-clot assay, a BLI assay carried out in accordance with an embodiment of the invention has a sensitivity of at least 0.05 EU/ml, with results in nearly half the time.

### EXAMPLE 4

This example demonstrates a variety of biosensor chemistries and different plates can be used in assays of samples in accordance with embodiments of the invention.

A biosensor having an SiO₂ functionalized tip is prepared as generally described in U.S. Patent 7,394,547, and a biosensor is having a Aminopropylsilane (APS) functionalized tip is prepared as generally described in U.S. Patent Application Publication 2010/0093106.

Endotoxin concentrations of 50, 5, 0.5, 0.05, 0.005, 0.0005, and 0 EU/mL are prepared. Lyophilized LAL reagent (Associates of Cape Cod Inc.; East Falmouth, MA) is resolubilized with endotoxin-free water and placed in contact with the endotoxin in wells of 96 well polystyrene plates, and 384 well polypropylene plates, agitated at 1000 rpm.

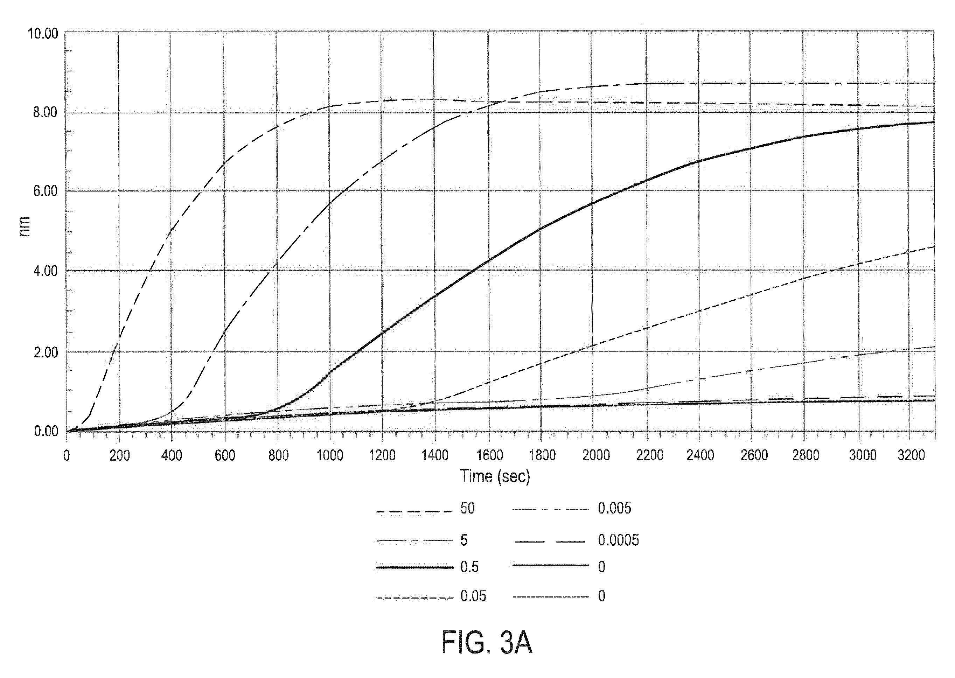

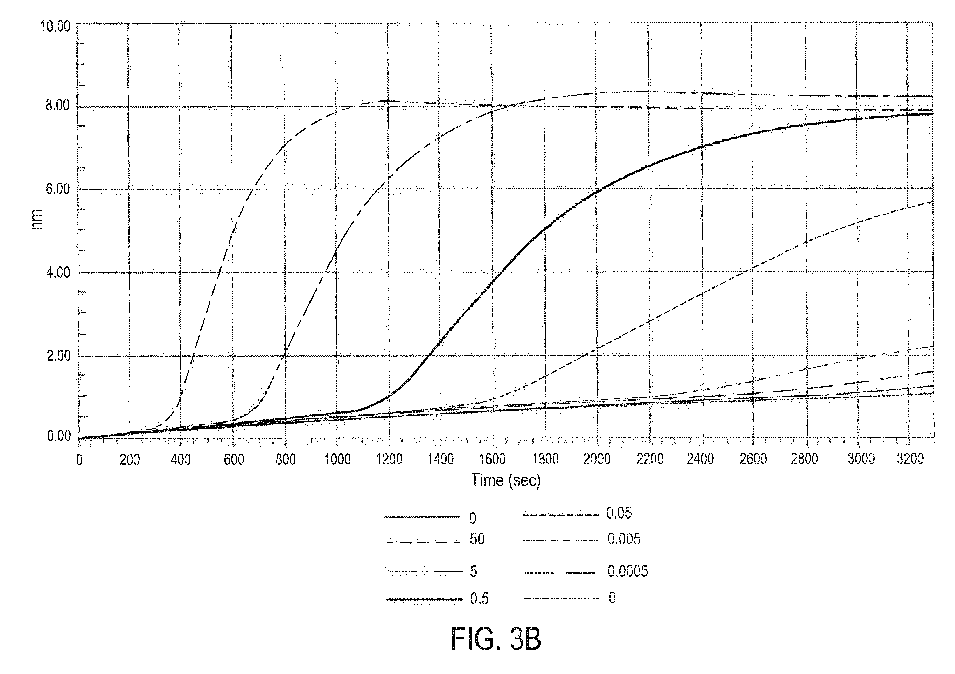

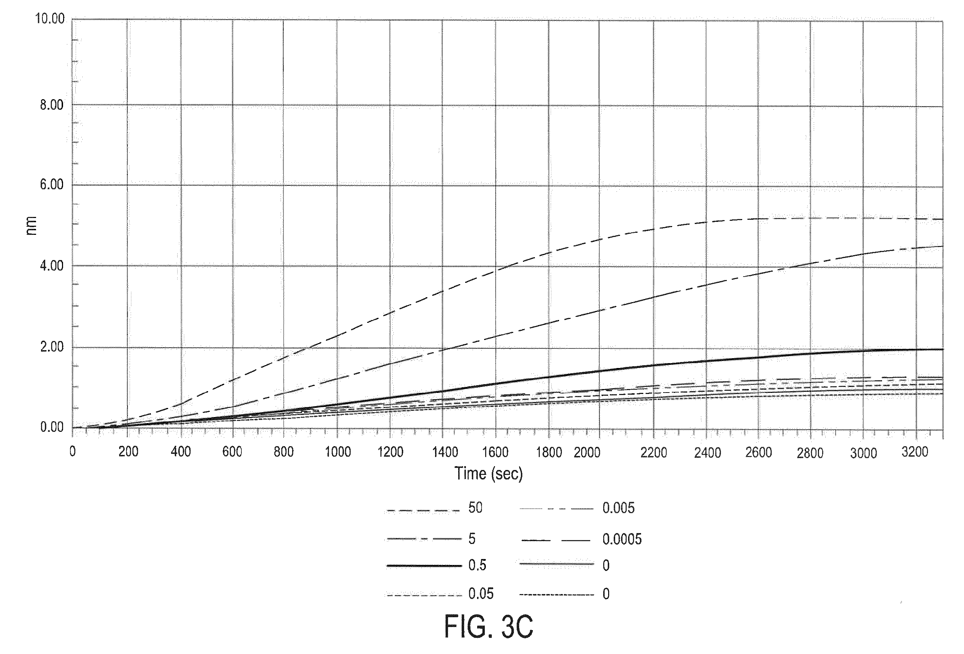

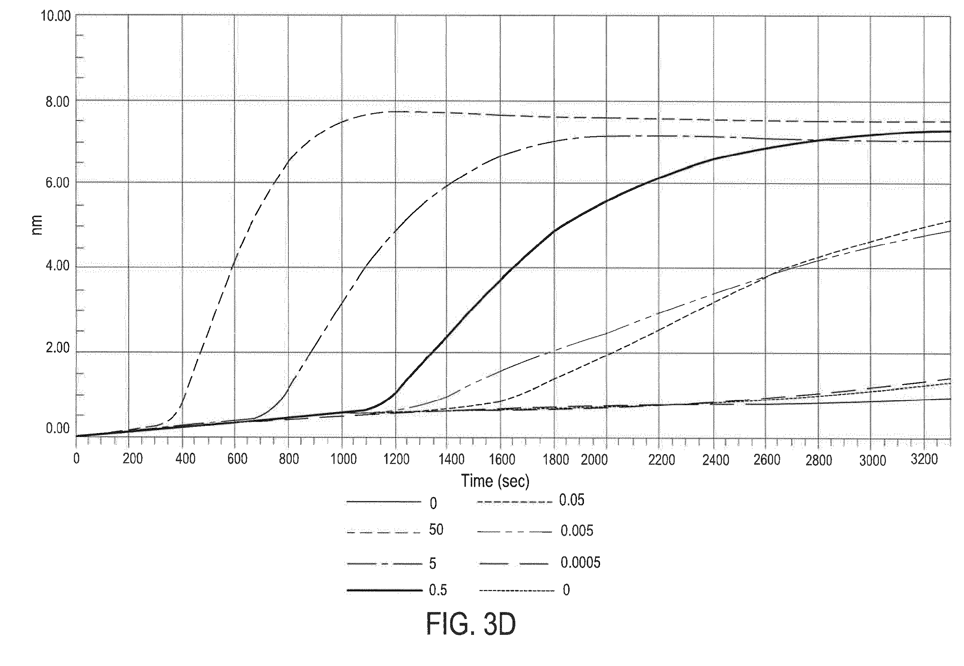

The samples are analyzed via BLI, and the results are shown in Figures 3A-3D.

This example shows, using polystyrene and polypropylene plates, endotoxin concentrations of 50, 5, and 0.5 EU/mL can be detected by both the SiO₂ functionalized tip and the APS functionalized tip biosensors. The biosensor with an APS functionalized tip can also detect endotoxin concentrations of 0.05, and 0.005 EU/mL using polystyrene and polypropylene plates.

### EXAMPLE 5

This example demonstrates a variety of endotoxin concentrations can be determined in assays of a sample with and without shaking in accordance with an embodiment of the invention.

A biosensor having an SiO₂ functionalized tip is prepared as generally described in U.S. Patent 7,394,547.

Endotoxin concentrations of 50, 5, 0.5, 0.05, 0.005, 0.0005, and 0 EU/mL are prepared. Lyophilized LAL reagent (Associates of Cape Cod Inc.; East Falmouth, MA) is resolubilized with endotoxin-free water and placed in contact with the endotoxin in wells of a 384 well polypropylene plate (the well reaction volumes are 40 µL; 4:1 ratio of sample to LAL), agitated at 1000 rpm (shaking) or 0 rpm (no shaking).

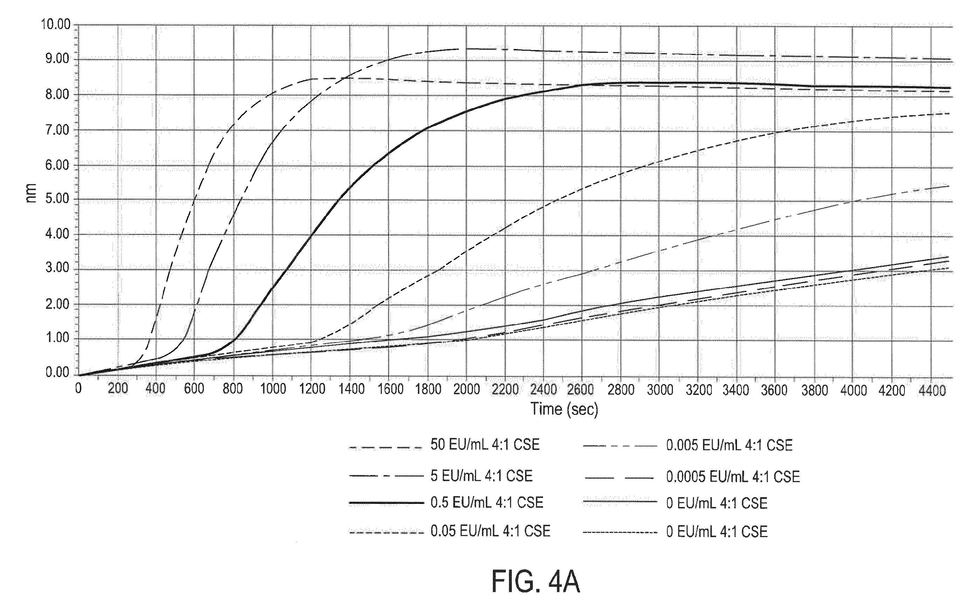

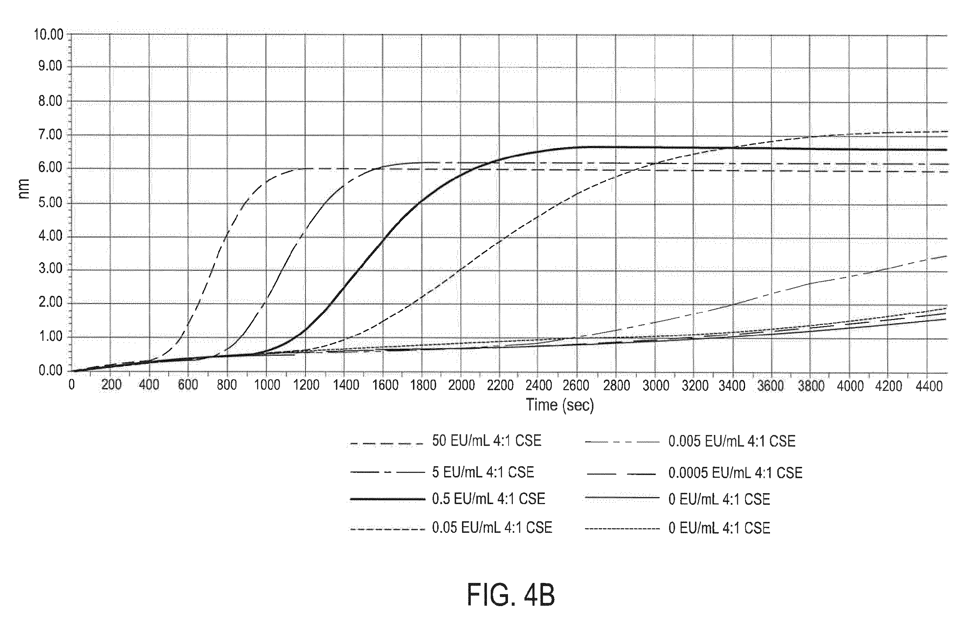

The samples are analyzed via BLI, and the results are shown in Figures 4A and 4B.

In this Example, no shaking slows the reaction down and decreases the signal, and lower concentrations are slowed more than higher concentrations. However, this example shows that endotoxin concentrations of 50, 5, 0.5, 0.05, and 0.005 EU/mL can be detected with and without shaking in accordance with embodiments of the invention.

### EXAMPLE 6

This example demonstrates that endotoxin concentrations can be determined in assays of a sample via BLI at different temperatures in accordance with an embodiment of the invention.

A biosensor having an SiO₂ functionalized tip is prepared as generally described in U.S. Patent 7,394,547. Endotoxin concentrations of 50, 5, 0.5, 0.05, 0.005, 0.0005, and 0 EU/mL are prepared. Assays are carried out at 33° C, 35° C, 37° C, and 41° C.

Endotoxin concentrations of 50, 5, and 0.5 EU/mL can be detected at all 4 temperatures. Endotoxin concentrations of 0.05 EU can be detected at 33° C, 35° C, and 37° C.

### EXAMPLE 7

This example demonstrates that endotoxin concentrations can be determined in assays of a sample via BLI with and without linear agitation (strokes per minute), in contrast with orbital agitation (at rpm) in accordance with an embodiment of the invention.

Endotoxin concentrations of 3000, 300, 30, 3, and 0 EU/mL are prepared for analyzing without linear agitation.

Endotoxin concentrations of 350, 35, 3.5, and 0.35 EU/mL are prepared for analyzing with linear agitation.

A biosensor having an APS functionalized tip is prepared as generally described in U.S. Patent Application Publication 2010/0093106.

The samples in this example are analyzed via BLI using the BLITZ™ system (fortéBIO® a division of Pall Life Sciences, Port Washington, NY), that can agitate samples using linear agitation.

One set of samples is agitated at 1200 strokes per minute, the other is not agitated.

The assays are carried out at 25° C, each agitated analyzed sample volume is 4 µL, and each non-agitated sample volume is 200 µL.

This example shows endotoxin concentrations can be detected in samples with and without linear agitation, and in particular shows endotoxin concentrations of 3000, 300, 30, and 3 EU/mL can be detected in an assay without agitation, and endotoxin concentrations of 350, 35, 3.5, and 0.35 EU/mL can be detected with linear agitation.

### EXAMPLE 8

Endotoxin concentrations of 50, 5, 0.5, 0.05, 0.005, 0.0005, and 0 EU/mL are prepared. A biosensor having an APS functionalized tip is prepared as generally described in U.S. Patent Application Publication 2010/0093106.

Set of samples are agitated at orbital agitation rates of 100 rpm, 1000 rpm, and 1400 rpm.

The samples are analyzed via BLI. At an agitation rate of 100 rpm, endotoxin concentrations of 50, 5, 0.5, and 0.05 EU/mL can be detected. At an agitation rate of 1000 rpm, endotoxin concentrations of 50, 5, 0.5, 0.05, and 0.005 EU/mL can be detected. At an agitation rate of 1400 rpm, endotoxin concentrations of 50, 5, 0.5, and 0.05 EU/mL can be detected.

This example demonstrates that endotoxin concentrations can be determined in assays of a sample at different rates of agitation in accordance with an embodiment of the invention.

The example also demonstrates that agitation is not necessary for carrying out analysis, though some agitation may decrease the clotting time, and faster agitation does not necessarily further speed up clotting.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of assaying a sample for endotoxin, the method comprising:
(a) placing a sample to be assayed in contact with LAL reagent, wherein endotoxin, if present in the sample, reacts with the reagent and a clotted product is formed;
(b) contacting a tip of an optical fiber with the sample, wherein clotted product, if present, forms on and/or binds to the tip of the optical fiber, wherein the tip of the optical fiber has an optical thickness, and the optical thickness increases from an initial thickness when clotted product forms on and/or binds to the tip of the optical fiber;
(c) determining the optical thickness at the tip of the optical fiber after contact with the sample, wherein an increase in optical thickness from the initial thickness indicates the presence of endotoxin, and an absence of an increase in optical thickness indicates the absence of endotoxin.

2. The method of claim 1, comprising determining a concentration of endotoxin in the sample.

3. The method of claim 1 or 2, comprising placing the sample in a sample receiving chamber, passing the sample from the sample receiving chamber to a reaction chamber having the LAL reagent therein, wherein the tip of the optical fiber contact the sample in the reaction chamber, and determining the optical thickness at the tip of the optical fiber after contact with the sample.

4. The method of any one of claims 1-3, including contacting the tips of a plurality of optical fibers with the sample, and determining the optical thicknesses at the tips of the optical fibers after contact with the sample.

5. The method of any one of claims 1-4, wherein a plurality of optical tips are arranged in a bundle, and the method includes contacting the tips of the plurality of optical fibers with the sample, and determining the optical thicknesses at the tips of the optical fibers after contact with the sample.

6. A kit for assaying a sample for endotoxin, the kit comprising a biosensor comprising an optical fiber having a tip, and a housing, the housing comprising a base and a cap, the base comprising a groove, and a reaction chamber having a side wall, the reaction chamber having an aperture passing through the side wall, the aperture communicating with the groove; the cap comprising a sample receiving chamber, and a projection including a sidewall and at least one aperture passing through the projection side wall, wherein the cap is removably engageable with the base.

7. The kit of claim 6, further comprising LAL reagent and a biosensor comprising an optical fiber having a tip, wherein the reaction chamber has LAL reagent therein, and the optical fiber is arranged in the groove of the base, wherein the optical fiber tip communicates with the reaction chamber through the aperture.

8. The kit of claim 6 or 7, including a plurality of biosensors and a plurality of reaction chambers, wherein each reaction chamber communicates with the tip of an optical fiber.
